# EUROPEAN PATENT APPLICATION

(11) **EP 4 286 008 A2**
(43) Date of publication of application: **06.12.2023**
(21) Application number: 23203339.9
(22) Date of filing: 24.06.2016
(51) Int. Cl.: A61P 35/00

(54) **METHOD OF TREATMENT OF NEUROENDOCRINE TUMORS THAT OVER-EXPRESS SOMATOSTATATIN RECEPTORS**

(30) Priority: 25.06.2015 US 201562176901 P
(62) Divisional of application: 16766600.7
(71) Applicant: Advanced Accelerator Applications, 01630 Saint Genis Pouilly (FR)
(72) Inventor: BUONO, Stefano, NEW YORK, 10118 (US); SIERRA, Maribel, Lopera, NEW YORK, 10118 (US)
(74) Representative: Plasseraud IP

(57) **Abstract**

The present invention relates to methods of treating cancers that over-express somatostatin receptors. More specifically, the invention provides a combined therapy in which a combination of Peptide Receptor Radionuclide Therapy (PRRT) and Immuno Oncology therapy (I-O therapy) are administered for the treatment of neuroendocrine tumors.

## Description

### RELATED APPLICATIONS

### [Not Applicable]

### FIELD OF THE INVENTION

The present invention relates to methods of treating cancers that over-express somatostatin receptors. More specifically, the invention provides a combined therapy in which a combination of Peptide Receptor Radionuclide Therapy (PRRT) and Immuno-Oncology therapies (I-O therapy) are administered for the treatment of neuroendocrine tumors.

### BACKGROUND OF THE INVENTION

Neuroendocrine neoplasia occurs in a variety of organ sites and tissue types. Neuroendocrine tumors (NETs) are tumors that arise from cells of the endocrine (hormonal) and nervous systems. Neuroendocrine tumors (NETs) include a group of tumors with a range of morphologic, functional, and behavioral characteristics. These tumors are generally slow growing. However, they have the potential to spread, primarily to the liver, and when they do, they can be life threatening and difficult to treat with current modalities.

NETs are rare, heterogeneous tumors originating from dispersed neuroendocrine cells that are distributed throughout the body. The term "neuroendocrine" relates to the ability of these cells to synthesize, store and secrete neuro-hormones, neuro-transmitters or neuro-modulators, which are produced by both the endocrine and the nervous systems.

Most patients with NETs do not exhibit symptoms and their tumors are discovered only upon unrelated surgery or exams. Although functioning NETs that produce certain hormones and other chemicals often cause patients to exhibit symptoms, the non-specific nature of these symptoms can lead to delayed diagnosis or even a misdiagnosis. By the time patients with NETs are correctly diagnosed, the cancer has usually metastasized, with regional or distant metastasis observed in approximately 50% of cases. Once they have metastasized, NETs cannot be effectively treated by surgery alone and generally are not curable.

Hence, there is a significant need for therapies for NETs, however, there are only a limited number of options currently available for the treatment of advanced NETs.

**The** cornerstone for treatment of NETs is the use of Somatostatin analogues (SSA) such as Sandostatin (Novartis) or Somatuline^{®} (Ipsen). Traditionally SSAs have been used for symptomatic treatment and, even if, some antitumoral activity has been confirmed. Chemotherapy and targeted therapies have been registered only for pancreatic NET (pNET), while for gastro-intestinal (GI) or pulmonary NETs only SSA have a limited approval of use. As a result of that, it is a fact that many patients exhaust all the available treatment options particularly in the advanced disease setting, which represents a high unmet medical need for systemic treatments of metastatic NETs.

At a symptomatic level, SSA are used to control certain clinical syndromes (collectively referred to as carcinoid syndrome) including severe diarrhea, flushing episodes and heart disease associated with metastatic carcinoid tumors.

Most NETs overexpress somatostatin receptors (SSTR). These protein G coupled receptors are expressed in human cells that sense molecules outside of the cell and activate sub-cellular events in response to the hormone somatostatin. This short-lasting endogenous hormone is an important regulator of the endocrine system, and somatostatin analogues that mimics its activity results in the inhibition of the activity of growth hormone, glucagon and insulin, have been found to be very effective in controlling the carcinoid's syndrome related symptoms. Growth hormone, glucagon and insulin often also are produced by carcinoid tumors.

However, the symptomatic treatment of carcinoid syndrome is distinct from treating the metastatic NETs themselves. Based on a partial antiproliferative effect, Ipsen's, Somatuline^{®} Depot^{®} (lanreotide), was approved by the FDA in December 2014 for the treatment of adult patients with unresectable, well-differentiated or moderately-differentiated, locally advanced or metastatic NETs in the gastro-entero-pancreatic tract (GEPNETs). Everolimus (Afinitor^{®}, an oral inhibitor of mammalian target of rapamycin (mTOR)) has been approved in the US for treatment of progressive neuroendocrine tumors of pancreatic origin, and well-differentiated non-functional, unresectable, locally advanced or metastatic NETs of gastrointestinal (GI) or lung origin (PFS of 11 months observed in Phase 3 trials). These are to date the only "antitumoral" therapy so far approved for midgut tumors. However, when the tumor becomes resistant or unresponsive to its effects, usually around 6-18 months after the initiation of treatment^{1,2,3}, there are no other approved therapies for intervention in GI and pulmonary NETs. These tumors therefore are untreatable by the currently available therapies.

While chemotherapeutic agents and some targeted therapies have become the standard of care for pNETs, the response rates vary according to tumor aggressiveness but are estimated to be between approximately 30% and 50%. Two targeted therapies have been approved for the treatment of progressive non-functioning pNETs, with limited survival benefit.

As discussed above in [0010], the first of these targeted therapies is the use of Afinitor^{®} (everolimus), a mammalian target of the rapamycin (mTOR) inhibitor, however, treatment with Afinitor^{®} may cause severe skin and gastrointestinal disorders, kidney and liver toxicity and bone marrow damage.

The only other treatment is the use of Sutent^{®} (sunitinib), a multi-targeted receptor tyrosine kinase inhibitor, or RTK, approved by the FDA and the EMA in 2011 for the treatment of unresectable or metastatic, well-differentiated pNETs with disease progression in adults on the basis of PFS of 11.4 months observed in Phase 3 trials. Again, this is a less than ideal intervention as treatment with Sutent^{®} may cause severe side effects such as renal failure, heart failure, gastrointestinal disorders, hemorrhages and hematological disorders (e.g., neutropenia, thrombocytopenia, and anemia), which are among its most common adverse drug reactions.

Thus, traditional chemotherapy has little place in treatment of well-differentiated NETs, since most of these tumors are slow growing and difficult to treat. A rigorous assessment of the efficacy of chemotherapy in literature is hampered by the prevalence of retrospective studies on heterogeneous series of patients, where toxicity is relevant, and the responses are short-lived and sporadic, particularly in "midgut carcinoids". Many schemes, including single or multiple agents, have been attempted.

Streptozotocin based schemes in pancreatic tumors yielded significant objective responses, but none of the schemes used in "midgut carcinoids" showed any activity in treating NETs. Some better initial responses have been reported for small populations for the combination of temozolomide plus capecitabine⁴ but again, these early data make it clear that the currently available therapies for NETs are inadequate and there is a long-felt need for additional robust therapies that can produce a significant therapeutic outcome.

The present invention, described in greater detail below, provides a combination therapy approach that effectively treats NETs.

### BRIEF SUMMARY OF THE INVENTION

The present invention relates to methods of treating a patient affected by a tumor that over-expresses somatostatin receptors comprising administering to said patient a combination of a Peptide Receptor Radionuclide therapy (PRRT) and an I-O therapy

In specific embodiments, the PRRT is [177]Lutetium-DOTA[O]-Tyr[3]-Octreotate.

In specific embodiments the I-O therapy comprises administering an inhibitor that inhibits the PD-1/PD-L 1 and CTLA-4 pathway. In other embodiments, the inhibitors of the PD-1/PD-L 1 and CTLA-4 pathway are antibodies that target the PD-1/PD-L 1 axis or the CTLA-4/B7 receptor complex. In still further embodiments, the inhibitor of the PD-1/PD-L 1 pathway is a combination of inhibitors that target both PD-1 and PD-L 1.

In particular embodiments, the PD-1 /PD-L1 or CTLA-4/B7 pathways are selected from the group consisting of Nivolumab, MK-3475, MPDL3280A, MED14736, ipilimumab, and tremelimumab.

The invention is particularly defined by embodiments in which the cancer is a neuroendocrine tumor. More specifically, said treating comprises one or more of inhibiting the growth of a neuroendocrine tumor, inhibiting proliferation of neuroendocrine tumor cells, inhibiting neuroendocrine tumor metastases, reducing tumorigenicity of neuroendocrine tumor cells and methods of reducing the frequency of cancer stem cells or tumor initiating cells in a neuroendocrine tumor.

Any neuroendocrine tumor that expresses or overexpresses somatostatin receptors may be treated by the methods of the present invention. Exemplary such tumors include but are not limited to the group consisting of a gastroenteropancreatic neuroendocrine tumor, carcinoid tumor, pheochromocytoma, paraganglioma, medullary thyroid cancer, pulmonary neuroendocrine tumor, thymic neuroendocrine tumor, a carcinoid tumor or a pancreatic neuroendocrine tumor, pituitary adenoma, adrenal gland tumors, Merkel cell carcinoma, breast cancer, Non-Hodgkin lymphoma, Hodgkin lymphoma, Head & Neck tumor, urothelial carcinoma (bladder), Renal Cell Carcinoma, Hepatocellular Carcinoma, GIST, neuroblastoma, bile duct tumor, cervix tumor, Ewing sarcoma, osteosarcoma, SCLC, prostate cancer, melanoma, meningioma, glioma, medulloblastoma hemangioblastoma, supratentorial primitive, neuroectodermal tumor, and esthesioneuroblastoma.

In particular examples, the neuroendocrine tumor is selected from the group consisting of functional carcinoid tumor, insulinoma, gastrinoma, vaso active intestinal peptide (VIP)oma, glucagonoma, serotoninoma, histaminoma, ACTHoma, pheocromocytoma, and somatostatinoma.

The neuroendocrine tumors treated by the present invention may be defined by grade, and may be low grade, medium grade, or high grade neuroendocrine tumors. In specific embodiments, the tumor is a functional neuroendocrine tumor. Alternatively, the neuroendocrine tumor is a non-functional neuroendocrine tumor.

In particularly preferred embodiments, the cancer is a small cell lung cancer. In other preferred embodiments, the cancer is a progressive midgut neuroendocrine tumor. In some embodiments, the invention provides therapy for such neuroendocrine tumors that are not responsive to Sandostatin (Novartis) or Somatuline^{®} (Ipsen). In other embodiments, the neuroendocrine tumor is non-responsive or has a low response to an inhibitor of the PD-1/PD-L 1 pathway.

### BRIEF DESCRIPTION OF SEVERAL VIEWS OF THE DRAWINGS

### [Not Applicable]

### DETAILED DESCRIPTION OF THE INVENTION

The present invention provides methods of inhibiting the growth of a neuroendocrine tumor, methods of inhibiting proliferation of neuroendocrine tumor cells, methods of treating or stabilizing a neuroendocrine cancer, methods of inhibiting neuroendocrine tumor metastases, methods of reducing tumorgenicity of neuroendocrine tumor cells and methods of reducing the frequency of cancer stem cells or tumor initiating cells in a neuroendocrine tumor. More specifically, the methods provided herein comprise administering a combination of Peptide Receptor Radionuclide with an immune-oncology therapy. In some embodiments, the Peptide Receptor Radionuclide Therapy (PRRT) is a therapy using a radiolabeled SSA peptide with high affinity for Somatostain receptors (sstr) that carries a radioactive isotope such as Lu-177 within its overall structure. In the present invention, the PRRT is combined with PD-1 and/or PD-L 1/CDLA-4 inhibitors.

### Lutathera:

SSAs are synthetic versions of Somatostatin endogenous hormone that have a longer half-life than the wild-type endogenous Somatostatin hormone. In specific embodiments, the preferred molecule used PRRT is Lutathera ([177]Lutetium-DOTA[O]-Tyr[3]-Octreotate), which is a ready-to-inject solution of a Lu-177-labeled-SSA.

Lutathera is comprised of three components. The first component is the SSA Octreotate. This component is the peptide that targets the NET cells. The second component is DOTA, a compound able to combine metals (such as Lu-177) into complexes through a ring structure; and the third component is Lu-177, a radioisotope.

Lutathera treats certain NETs by selectively binding to SSTR2 receptors, the most commonly expressed receptors on these types of tumors. Lutathera then destroys NET cells in a targeted fashion by delivering a local emission of high-energy electrons. Because Lutathera also emits gamma radiation, it can also be useful as a disease management tool, as this kind of emission can be captured with a SPECT camera and thus be used for determining the drug's distribution and pharmaco-kinetics and also for dosimetric estimations.

In light of the current limited options and effectiveness for treatment of NETs overall and the lack of treatments for progressive midgut NETs specifically, Lutathera can meet a significant medical need by potentially improving patient outcomes in the treatment of progressive midgut NETs, as well as other somatostatin-receptor-positive tumors.

Lutathera is the first ever PRRT (Peptide Receptor Radionuclide Therapy) radiopharmaceutical product being tested in a Phase 3 trial for the treatment of inoperable, progressive, well-differentiated, SSTR2-positive, midgut NETs.

The current Phase 3 trial is a multi-center, randomized, comparator-controlled, parallel-group study evaluating the efficacy and safety of Lutathera (using total cumulative administered radioactivity of 29.6 GBq) compared to Sandostatin^{®} LAR 60 mg.

### Immuno-Oncology therapies

Immuno-Oncology therapy (or I-O therapy) is an emerging field of cancer treatment that utilizes the body's own immune system to fight diseases. The goal of I-O therapy is to restore the ability of the immune system to eliminate cancer cells by either activating the immune system directly, or by inhibiting mechanisms of suppression by tumors.

The immune system depends on multiple checkpoints or "immunological brakes" to avoid overactivation of the immune system on healthy cells^{5.6}. Down regulating the immune system by preventing the activation of T-cells, reduces autoimmunity and promotes self-tolerance. Tumor cells often take advantage of these checkpoints to escape detection by the immune system. CTLA-4 and PD-1 are checkpoints that have been studied as targets for cancer therapy^{5·6}.

Programmed cell death protein 1, also known as PD-1 is a cell surface receptor that is expressed on T cells. PD-1 binds PD-L 1, which is expressed on a variety of cells in the body, including many tumour cells; it inhibits T-cell function contributing to the tumor's ability to evade the immune system ^{5·6}.

Cytotoxic T-lymphocyte-associated antigen 4 (CTLA-4; also known as CD152) is expressed on the surface of T cells, where it primarily suppresses their early stages of activation by inducing inhibitory downstream T-cell receptor (TCR) signaling and counteracting activity of the T-cell costimulatory receptor, CD28.^{19,20}[CTLA-4 is thought to outcompete CD28 for B7 ligands (CD80 and CD86) on the surface of antigen-presenting cells by binding them with higher affinity and avidity.²¹In preclinical studies, blockade of CTLA-4 led to a 1.5-fold to 2-fold increase in T-cell proliferation and a 6-fold increase in interleukin-2 production.²²

The physiologic role of CTLA-4 is not only to suppress effector T cells (Teffs), but also to increase the function of immunosuppressive CD4+FoxP3+ regulatory T cells (Tregs). Treg-specific CTLA-4 deficiency was shown to diminish the suppressive capacity of Tregs in cell culture, resulting in upregulation of CD80 and CD86 expression on dendritic cells (DCs).²³CTLA-4 blockade has been shown to promote T-cell activation, and in preclinical models, to deplete intratumoral Tregs in a process dependent on the presence of Fcγ receptor-expressing macrophages within the tumor microenvironment.^{24,25}

On March 25, 2011, the U. S. Food and Drug Administration approved ipilimumab injection (YERVOY, Bristol-Myers Squibb Company) for the treatment of unresectable or metastatic melanoma. The approval was based on a randomized (3:1:1), double-blind, double-dummy clinical trial (MDX010-20) in patients with unresectable or metastatic melanoma who had received at least one prior systemic treatment for melanoma. Overall survival (OS) was the trial's primary endpoint. Progression-free survival and best overall response rate were also assessed.

Ipilimumab was granted a marketing authorisation in the European Union (EU) on 13 July 2011 and is currently approved for the indication "treatment of advanced (unresectable or metastatic) melanoma in adults who have received prior therapy". Pursuant to Article 16 of Commission Regulation (EC) No 1234/2008, Bristol-Myers Squibb Pharma EEIG submitted to the European Medicines Agency on 2 August 2012 an application for a variation including an extension of indication. In October 2013, the MAH proposed the extension of indication of Yervoy for the treatment of previously untreated adult patients with advanced (unresectable or metastatic) melanoma.

Ipilimumab has been evaluated in combination with RT in patients with metastatic CRPC and melanoma. Promising activity with manageable tolerability was observed in a phase I/II trial in patients with CRPC who had progressed after anti-androgen therapy²⁶; however, results from a phase III trial showed no significant improvement in OS with the addition of ipilimumab to RT in pos-tdocetaxel CRPC. A subgroup analysis did suggest benefit for patients with less advanced disease²⁷. An analysis of clinical data from 21 patients with advanced melanoma who had received RT after ipilimumab progression on the Italian Expanded Access Program indicated that RT after ipilimumab treatment may further potentiate its effect²⁸ A local response to RT was detected in 13 patients (62%), while 8 patients (38%) did not show any local regression. The median OS for all 21 patients was 13 months (range 6-26). Eleven (85%) out of 13 patients with local response showed an abscopal effect, suggesting that local response to RT may be predictive for the abscopal response and outcome. The median OS for patients with and without abscopal responses was respectively of 22.4 months (range 2.5-50.3) and 8.3 months (range 7.6-9.0). There are now over 15 clinical trials alone in progress to evaluate ipilimumab plus RT.

Both PD-1 and PD-L 1 inhibitors target the interaction between PD-1 and PD-L1. The interaction between PD-L 1 and PD-1 results in dampening an immune response. Inhibiting a checkpoint like PD-1 or PDL-1 "releases the brakes" of the immune system and may enhance the anti-tumor T-cell response. This class of therapy has shown efficacy in cancer. Compared to other previously tested immunotherapies, PD-1/PD-L 1 inhibitors, when used as monotherapies, appear to shrink tumors in a much higher proportion of patients, across a wider range of tumor types and with a lower rate of high-grade toxicities, mainly immune-mediated side-effects. Compared with standard therapies, including chemotherapy and "targeted" therapies, immunotherapy, including PD-1/PD-L 1 inhibitors, appears to result also in a longer duration of response.

Nivolumab (Opdivo, Bristol-Myers Squibb) targeting PD-1 receptors was approved in Japan in July 2014 and by the US FDA in December 2014 to treat metastatic melanoma (second line). In April 2015 it obtained a Positive Opinion at EMA for approval as a monotherapy for metastatic melanoma. It is currently investigated in non-small cell lung cancer (NSCLC, squamous 3rd line) and Hodgkin lymphoma (3rd line).

Some tumor types have shown little response to anti-PD-1/PD-L 1 monotherapy in early clinical trials, including prostate, colorectal and pancreatic cancers. For such diseases, as well as for non-responders within the group of more immunoresponsive tumor types, combination therapy may help elicit an immune response. No clinical trials have ever been proposed for Neuro Endocrine Tumours.

PD-L 1 and PD-1 protein expression were analyzed in 94 clinical cases of small cell carcinomas ⁷. None of the small cell carcinomas showed PD-L 1 protein expression in tumor cells. PD-L 1 and PD-1 expression was noticed in the stroma: using immunohistochemistry, 18.5% of cases showed PD-L 1 expression in tumor-infiltrating macrophages and 48% showed PD-1 positive lymphocytes. RNA-seq showed moderate PD-L 1 gene expression in 37.2%. PD-L 1 was correlated with macrophage and T-cell markers. The second PD-1 ligand PD-L2 was expressed in 27.9% and showed similar correlations. Thus, the PD-1/PD-L 1 pathway seems activated in a fraction of small cell carcinomas. Other experience from the Charite' Hospital (Berlin) and the Zentrlklinic from Badberka (Germany) ⁸, suggests also higher CD3 positive lymphocytes presence and PD-L 1 expression in Poorly differentiated NETs compared to Well differentiated ones. Therefore, authors concluded indicating that PD-1 / PD-L 1 may be promising targets for immunotherapy especially in poorly differentiated NETs.

Nevertheless, patients with PD-L 1 IPD-L2 expression may respond to anti-PD-1 treatment. PD-L 1 expression by the tumor, however is not a perfect, predictor of response to PD-1/PD-L 1 inhibitors when they are used as monotherapies. In fact, PD-L 1 negative tumors can still respond in a lower percentage, while not all PD-L 1 positive tumors respond, although they are more likely to respond.

Small cell lung cancer and extrapulmonary small cell carcinomas are the most aggressive type of neuroendocrine carcinomas (G3), also called Poorly Differentiated Neuroendocrine Carcinomas (PDNEC). No study has been done on the PD-1/PD-L 1 pathway on more indolent forms of Neuro Endocrine Tumors such as G1 and G2 (also called well or moderately differentiated tumors) originating in other organs.

Antonia et al [AdRef.11] reported preliminary data at ASCO 2014 on 49 patients treated with the combination of nivolumab plus ipilimumab. Patients received either nivolumab 1 mg/kg plus ipilimumab 3 mg/kg, or nivolumab 3 mg/kg plus ipilimumab 1 mg/kg for 4 cycles, followed by nivolumab alone. Treatment-related, grade 3-4 AEs occurred in 49% of patients and 35% of patients discontinued study drugs due to treatment-related AEs. The most common grade 3-4 treatment-related AEs included diarrhea (n = 5, 10%), ALT elevation (n = 4, 8%), AST elevation (n = 4, 8%), colitis (n = 4, 8%), lipase elevation (n = 4, 8%), fatigue (n = 3, 6%), and pneumonitis (n = 3, 6%). The discontinuation rate due to drug-related AEs was highest in the nivolumab-1-mg/kg/ipilimumab 3-mg/kg-nonsquamous arm, and most discontinuations occurred during the concurrent phase of treatment. The response rate ranged from 11% to 33%, with some patients having prolonged duration of response.

Antonia et al [AdRef.11] reported at ASCO 2015 on the combination of tremelimumab plus MEDI4736. Of 102 patients treated with MEDI4736-plus-tremelimumab combination therapy in the dose-escalation phase, 40% had grade 3 or higher treatment-related AEs, with the most common treatment-related, grade 3-4 events being colitis (n = 9, 9%), diarrhea (n = 8, 8%), pneumonitis (n = 4, 4%), AST increase (n = 4, 4%), and ALT increase (n = 3, 3%). The observed response rate was 27% across all cohorts, with a 41% rate exhibiting disease control (CR, PR, and SD of at least 16 weeks).^{28,29}

Recent studies suggest that radiotherapy can be applied as a powerful adjuvant to immunotherapy and, in fact, can contribute to convert the irradiated tumor into an in situ vaccine, resulting in specific immunity against tumors cells ^{9,10}.

However, the clinical observation that best demonstrates the induction of antitumor immunity by external radiotherapy is the abscopal effect (that refers to a regression of non-irradiated lesions in patients treated with concurrent radiotherapy). Although it has been reported in multiple tumor types over the years, abscopal effects are infrequent ^{9,}. Their uncommon occurrence reflects existing barriers to successful immunization by radiotherapy.

The optimal radiation regimens to be used to harness the proimmunogenic effects of radiation remain to be defined. The synergistic effects of external radiation and checkpoint inhibitor therapy have been tested in various studies. However it seems that fractionation and dose play a major role in the induction of synergistic effect of radiation and checkpoint inhibitor treatment ¹¹.

Even if fractionated, the effect of external radiotherapy in the increasing of the antitumor immunity, is temporary.

This drawback could be surprisingly solved by the use of peptide receptor radionuclide therapy.

Thus the present invention relates to a novel combination of PRRT and I-O, which synergistically could decrease cancer cell growth without increasing the toxicity profile compared to the individual drugs.

PRRT is able to induce an anti-tumor immune response in addition to its ability to disrupt tumor cell cycling through delivery of ionizing radiation. For example it was observed that treatment with Lutathera PRRT in human xenograft tumor model of NET results in increased CD86+ APC infiltration and increased expression of CD86 on these cells ¹².

Due to the receptor mediated mechanism of action, the PRRT agent allows a continuous, stable internal radiotherapy, resulting in increased, constant and durable antigenic exposure.

In this context, the pro-immunogenic effects of radiotherapy can be enhanced by the use of PRRT to correct the immunosuppressive networks that are in place once tumors have established. Modern I-O agents such as anti PD-1/PD-L 1/CTLA-4 are gradually taking on the challenge of defeating the established tolerance toward the cancer, to recover an effective tumor-specific immune response. The proposed combination induces cell death via a novel mechanism: internal radiation to the cells using a metabolic pathway (the over-expression of somatostatin receptors by the tumor) and creating damage to the tumors that releases tumor antigens, making itself more visible to the immune system. The immunotherapy, such as immune checkpoint blockade (for e.g., the inhibition of the PD-1/PD-L 1/CTLA-4 pathway) should improve the immune anti-tumor T-cell response and enhance the effect of the radiation, in a synergistic unexpected way.

The use of PRRT with SSA agonists could be of additional advantage for a durable response based on their recognized stable internalization during the whole therapy, while preserving healthy tissues. For this aim, the use of specific PRRT that have the property to be internalized by the tumor cells could be preferred (for e.g. Lutathera).

Lymphocyte toxicity is commonly observed after PRRT ^{13 14}. This toxicity is the main factor preventing physicians and oncologists to suggest the combination of PRRT and I-O. For example, Denoyer et al (2015) noted that Lu-DOTATATE has toxicity against peripheral blood lymphocytes. There is general acknowledgement that both T and B lymphocytes have a crucial role in the immunoresponse to cancer (Linnebacher et al.). However, the present inventors also have shown that the toxicity of Lutathera is more specific to B-lymphocytes¹⁶. This finding is important as T- lymphocytes are the most abundant tumor infiltrates, and hence most implicated in the fight against cancer.

Lymphocytopenia after PRRT, which might reduce the immunologic response, is more severe with the Y90 radionuclide rather than Lu177¹⁶.

Bodei et al from the group of Milan³¹ (Bodei L, et al, 2014) presented a retrospective analysis of toxicity after PRRT in 807 patients treated from 1997 to 2013 with 177Lu-based PRRT, 90Y-based PRRT or the two compounds combined. Severe hematologic toxicity Grade 3-4 was observed in 14.1% of the cases treated with 90Y PRRT and 3.1 with 177Lu,, showing the more favorable hematological safety profile of PRRT with Lu-DOTATATE (Lutathera) compared to Y90.

Finally, the toxicity profile of the two treatments (PRRT and I-O) is different so they are not expected to cause significant increased side effects to patients.

For example, as the administration of Lutathera could lead to hematotoxicity essentially affecting B cells and not T cells, Lutathera should not affect the immuno response of the anti-PD1/PDL 1/CTLA-4 treatments which is based on T cells. In view of the above, it is contemplated that a combination of Lutathera with an inhibitor that inhibits the PD-1/PD-L 1/CTLA-4 pathway will provide an effective and new treatment for NETs which have traditionally been difficult to treat.

This combined effect should occur in every cancer over-expressing somatostatin receptors. The combination should prolong the effects of PRRT (for e.g. Lutathera) on Grade I-II NET (the indication where Lutathera is considered appropriate), as well as allowing a significant therapeutic effect also on those cancers that have not shown significant responses to Lutathera in Phase I-II clinical studies or Proof of Concept studies, such as Grade III NET. Small cell lung cancer and extrapulmonary small cell carcinomas being the most aggressive type of neuroendocrine carcinomas and could benefit from this combination with unexpected results.

In support of this theory, some studies using with combination of PRRT with other kind of agents, mostly chemotherapy, have shown very good results.

The co-expression of sstr2 and PD-1/PD-L1, and/or PD-L2 receptors on some neuroendocrine tumour tissues overexpressing sstr2 has been assessed by immunohistochemistry. Human tumor tissue samples from small cell lung cancer and colorectal cancer (both neuroendocrine) were used for this evaluation. The relationship between sstr2 expression and CTLA-4 is less well documented.

Formalin-fixed paraffin embedded (FFPE) tissue sections (5-6 µm thick) were used. The sections were dried and de-paraffinated. Immunohistochemistry (IHC) staining was performed using Anti-PD-1 (diluted 1:100), Anti-PD-L1 (diluted 1: 50) and Anti-SSTR2 (diluted 1:100) primary antibodies from Thermo Fisher. Anti-rabbit and anti-mouse secondary antibodies from ImmunoCruz LSAB Staining System were used. A standard procedure for IHC staining was followed, with an incubation time for the primary antibody of 1 h 45 min. For the secondary antibody the incubation time was 30 min. At the end of the IHC procedure the tissues were de-hydrated and images acquired with Olympus BX60 equipped with Leica DFC420C and Leica LAS software.

Preliminary results of the IHC staining indicate the expression, in both tissue types, of PD-1 and sstr2 receptors. In the tissue specimen analyzed, the PD-L1 appears to be expressed at a lower extent, but further analyses are ongoing.

Additional immunohistochemistry analyses and mRNA assay on tissue samples from other sstr2-expressing tumors are ongoing to further support the rationale of the present invention. Moreover, additional exploratory pre-clinical tests are planned, to assess the efficacy of a combination therapy with 177Lu-DOTATATE and anti-PD1 (or anti-PDL-1 and CTLA-4) antibody in a suitable rat pancreatic model of neuroendocrine tumor.

### Methods of Treatment

The present invention provides methods of treating neuroendocrine tumors. Neuroendocrine tumors (NETs) are tumors that arise from cells of the endocrine (hormonal) and nervous systems. Neuroendocrine tumors (NETs) include a group of tumors with a wide range of morphologic, functional, and behavioral characteristics. These tumors are generally slow growing but have the potential to spread, primarily to the liver, and when they do, they can be life threatening and difficult to treat with current modalities.

Neuroendocrine tumors have traditionally been classified by the site of their origin. In certain embodiments, the NET is selected from the group consisting of pancreatic neuroendocrine tumors (pNETs) and carcinoid tumors of the lung, stomach, duodenum, jejunum, ileum, colon and rectum. In further embodiments, the NET is selected from the group consisting neuroendocrine tumors of the ovary, thymus, thyroid medulla, adrenal glands (e.g., pheocromocytoma) and paraganglia (paraganglioma). In certain embodiments, the NET treated by the methods described herein is small cell lung cancer (SCLC). In certain alternative embodiments, the NET is a non-small cell lung cancer. In certain embodiments, NETs are pancreatic neuroendocrine tumors (PETs) or carcinoid tumors. In certain embodiments, the NET is non-small cell lung cancer, a pancreatic cancer, or a thyroid cancer.

Neuroendocrine tumors are also classified by grade and differentiation. See, e.g., Phan et al., Pancreas, 39(6):784-798 (2012) ¹⁵. In certain embodiments, the neuroendocrine tumor is a well differentiated, low grade tumor. In certain embodiments, the neuroendocrine tumor is a moderately differentiated, intermediate grade tumor. In certain embodiments, the neuroendocrine tumor is a poorly differentiated, high grade tumor. In one embodiment, low grade tumors are characterized by <2 mitoses per 10 HPF (high power fields) and no necrosis. In one embodiment, intermediate grade tumors are characterized by 2-10 mitoses per 10 HPF (high power fields) or foci of necrosis. In one embodiment, high grade tumors are characterized by >10 mitoses per 10 HPF (high power fields).

In other embodiments neuroendocrine tumors can be divided based on the WHO classification 2000 and 2010 into Neuroendocrine tumours Grade 1 -Grade 2 (or Well-differentiated endocrine tumour or carcinoma (WDET / WDEC), Neuroendocrine carcinoma Grade 3 or Poorly differentiated endocrine carinoma/small-cell carcinoma (PDEC), Mixed adenoneuroendocrine carcinoma (MANEC) and Hyperplastic and preneoplastic lesions. According to the ENETSIWHO/AJCC Classification systems Tumors G1 are those with Ki67 index ≤ 2% or MI (mitotic count) <2, Tumors G2 are those with Ki67 index within 3 -20% or MI = 2-20 and tumors G3 are those with Ki67 index ≥ 20% or MI >20.

Neuroendocrine tumors are also classified as functional and non-functional NETs. NETs are considered functional when a specific clinical syndrome is induced due to excessive production of hormones by the tumor cells. Examples of functional NETs include, but are not limited to, carcinoid tumors, which can result in carcinoid syndrome, and functional pNETs, for example, insulinomas, gastrinomas, vasoactive intestinal peptide (VI P)omas, glucagonomas, and somatostatinomas.

Non-functional NETs are not associated with a clinical syndrome due to excessive production of hormones by the tumor cells, but can still produce symptoms related to the presence of the tumor or its metastasis (e.g., abdominal pain or bloating). In certain embodiments, the neuroendocrine tumor is a functional NET. In certain embodiments, the neuroendocrine tumor is a non-functional NET. In certain embodiments, the neuroendocrine tumor is selected from the group consisting of functional carcinoid tumor, insulinoma, gastrinoma, vaso active intestinal peptide (VI P)oma, glucagonoma, serotoninoma, histaminoma, ACTHoma, pheocromocytoma, and somatostatinoma. In certain embodiments, the neuroendocrine tumor is NSCLC.

In certain embodiments, the neuroendocrine tumor is a primary tumor. In alternative embodiments, the neuroendocrine tumor is metastatic tumor. In certain embodiments, the neuroendocrine tumor has not spread outside of the wall of the primary organ. In certain embodiments, the neuroendocrine tumor has spread through the wall of the primary organ and to nearby tissues, such as fat, muscle, or lymph nodes. In certain embodiments, the neuroendocrine tumor has spread to tissues or organs away from the primary organ, for example, to the liver, bones, or lungs.

In specific embodiments, it is contemplated that the methods of the present invention will be particularly useful in the treatment of neuroendocrine cancer or tumor that is refractory to treatment. As a non-limiting example, the cancer or tumor may be chemorefractory (i.e., resistant to one or more forms of chemotherapy). In certain embodiments, the cancer or tumor is resistant to treatment with a somatostatin analog. In certain embodiments, the cancer or tumor is resistant to treatment with a kinase inhibitor. In still other embodiments the cancer or tumor is resistant to treatment with an inhibitor of the PD-1/PD-L 1/CTLA-4 pathway.

In certain embodiments, the neuroendocrine cancer or tumor has metastasized to the liver. By way of non-limiting example, the neuroendocrine cancer or tumor is a carcinoid or pancreatic neuroendocrine tumor that has metastasized to the liver.

In one aspect, the present invention provides the use of a combination of PPRT with I-O therapy in the treatment of a neuroendocrine tumor. This combination of treatments is useful for inhibiting neuroendocrine tumor growth, reducing neuroendocrine tumor volume, and/or reducing the tumorigenicity of a neuroendocrine tumor. The methods of use can be in vitro, ex vivo, or in vivo methods. In certain embodiments, the PPRT is Lutathera. PRRT is a kind of targeted radionuclide therapy, that is, a form of treatment that delivers therapeutic doses of radiation to malignant tumors, for example, by administration of a radiolabeled molecule designed to seek out certain cells. In certain embodiments, the PPRT is Lutathera. A non-exhaustive list of other agents that can be used for PRRT include but are not limited to ¹¹¹In-DTPA-octreotide, ⁹⁰Y-DOTATOC, ⁹⁰Y-DOTATATE, ¹⁷⁷Lu-DOTATOC, ⁹⁰Y-Lanreotide, ¹⁷⁷Lu-DOTACIN, ¹¹¹In-DOTA-BASS, ¹⁷⁷Lu-DOTA-JR11, and any therapeutic agent using SSA peptides radiolabeled with unsealed radiolabelled sources such as (but not limited to) ¹³¹Iodine, ¹⁵³Samarium, ²²³Radium, ²²⁵Actinium/²¹³Bismuth, ²¹¹Astatine, ¹⁶⁶Holmium, ¹⁸⁶Rhenium, ¹⁸⁸Rhenium, 67Copper, ¹⁴⁹Promethium, ¹⁹⁹Gold, ¹⁰⁵Rhodium, ⁷⁷Bromine, ¹¹¹Indium and ^{123/125} Iodine.

Lutathera may be produced by methods well known to those of skill in the art. Exemplary such methods include those described in US 5,804,157 or US 5,830,431.

The present invention provides for methods of treating neuroendocrine tumor comprising administering a therapeutically effective amount of a PPRT in combination with an inhibitor of the PD-1/PD-L 1/CTAL-4 pathway to a subject (e.g., a subject in need of treatment). In certain embodiments, the neuroendocrine tumor is a pancreatic neuroendocrine tumor. In certain embodiments, the neuroendocrine tumor is a carcinoid. In certain embodiments, the neuroendocrine tumor is neuroendocrine tumor of the lung. By way of non-limiting example, the neuroendocrine tumor in the lung may be SCLC. The invention is particularly useful for the treatment of neuroendocrine tumors that overexpress SSTR in their cellular surface such as (but not limited to) pituitary adenomas, gastrointestinal and pancreatic endocrine carcinomas (GEPNET tumours), pulmonary NET, paragangliomas, pheochromocytomas, small cell lung cancers, medullary thyroid carcinomas, breast cancers, prostate cancer and malignant lymphomas. In certain embodiments, the subject is a human. In certain embodiments, the PPRT is Lutathera.

The present invention further provides methods for inhibiting neuroendocrine tumor growth using a therapeutically effective amount of a PPRT in combination with an inhibitor of the PD-1/PD-L 1/CTLA-4 pathway to a subject. In certain embodiments, the method of inhibiting the neuroendocrine tumor growth comprises contacting the tumor cell with a therapeutically effective amount of one or both of a PPRT and with an inhibitor of the PD-1/PD-L 1/CTLA-4 pathway to a subject in vitro. For example, an immortalized neuroendocrine tumor cell line is cultured in medium to which is added the PPRT to inhibit tumor growth. In some embodiments, neuroendocrine tumor cells are isolated from a patient sample such as, for example, a tissue biopsy, pleural effusion, or blood sample and cultured in medium to which is added the PPRT and/or the inhibitor of the PD-1/PD-L 1/CTLA-4 pathway to inhibit tumor growth.

In some embodiments, the method of inhibiting neuroendocrine tumor growth comprises contacting the neuroendocrine tumor or tumor cells with combination therapy of the present invention in vivo. In certain embodiments, contacting a neuroendocrine tumor or tumor cell with a PPRT and an inhibitor of the PD-1/PD-L 1/CTLA-4 pathway is performed in an animal model. For example, a PPRT and an inhibitor of the PD-1/PD-L 1/CTLA-4 pathway may be administered to neuroendocrine tumor xenografts that have been grown in immunocompromised mice (e.g. NOD/SCID mice) to inhibit neuroendocrine tumor growth. In some embodiments, neuroendocrine tumor cancer stem cells are isolated from a patient sample such as, for example, a tissue biopsy, pleural effusion, or blood sample and injected into immunocompromised mice that are then administered the combination therapy of the present invention (i.e., a PPRT and an inhibitor of the PD-1/PD-L 1/CTLA-4 pathway) to inhibit neuroendocrine tumor cell growth. In some embodiments, the PPRT and/or the PD-1/PD-L 1/CTLA-4 pathway inhibitor is administered at the same time or shortly after introduction of tumorigenic cells into the animal to prevent neuroendocrine tumor growth. In some embodiments, the PPRT and/or the inhibitor of the PD-1/PD-L 1/CTLA-4 pathway is administered as a therapeutic after the tumorigenic cells have grown to a specified size.

In certain embodiments, the method of inhibiting neuroendocrine tumor growth comprises administering to a subject a therapeutically effective amount of the PPRT and the inhibitor of the PD-1/PD-L 1/CTLA-4. In certain embodiments, the subject is a human. In certain embodiments, the subject has a neuroendocrine tumor or has had a tumor removed.

In certain embodiments, the neuroendocrine tumor is a pancreatic neuroendocrine tumor. In certain embodiments, the neuroendocrine tumor is a carcinoid. In certain embodiments, the neuroendocrine tumor is neuroendocrine tumor of the lung. In certain embodiments, the neuroendocrine tumor is NSCLC.

In addition, the invention provides a method of reducing the tumorigenicity of a neuroendocrine tumor in a subject, comprising administering a therapeutically effective amount of a PPRT and an inhibitor of the PD-1/PD-L 1 to the subject. In certain embodiments, the neuroendocrine tumor comprises cancer stem cells. In certain embodiments, the frequency of cancer stem cells in the neuroendocrine tumor is reduced by administration of the agent. In certain embodiments, the PPRT is Lutathera.

Thus, the invention also provides a method of reducing the frequency of cancer stem cells in a neuroendocrine tumor, comprising contacting the tumor with an effective amount of a PPRT and an inhibitor of the PD-1/PD-L 1/CTLA-4 pathway.

As noted herein, the PPRT is administered in combination with an inhibitor of the PD-1/PD-L 1 pathway. In such methods, the PPRT may be administered prior to, concurrently with, and/or subsequently to administration of the PD-1/PD-L 1/CTLA-4 inhibitor. Pharmaceutical compositions comprising the PPRT and the PD-1/PD-L 1/CTLA-4 inhibitor are also provided. It is contemplated that the combined treatment with the PPRT and the PD-1/PD-L 1/CTLA-4 inhibitor has a synergistic effect on the treatment of the NETs.

It will be appreciated that the combination of a PPRT and the PD-1/PD-L 1/CTLA-4 inhibitor agent may be administered in any order or concurrently. In selected embodiments, the PPRT and PD-1/PD-L 1/CTLA-4 will be administered to patients that have previously undergone treatment with other anti-cancer agents. In certain other embodiments, the PPRT and the PD-1/PD-L 1/CTLA-4 inhibitor agent will be administered substantially simultaneously or concurrently. For example, a subject may be given PPRT while undergoing a course of treatment with the PD-1/PD-L 1/CTLA-4 inhibitor agent. In addition it is contemplated that the subject has already or may be concurrently receiving other forms of cancer therapy, e.g., chemotherapy. In certain embodiments, the PPRT will be administered within 1 year of the treatment with the PD-1/PD-L 1/CTLA-4 inhibitor agent. In certain alternative embodiments, the PPRT will be administered within 10, 8, 6, 4, or 2 months of any treatment with the PD-1/PD-L 1/CTLA-4 inhibitor agent and/or additional anti-cancer agent. In certain other embodiments, the PPRT will be administered within 4, 3, 2, or 1 week of any treatment with the PD-1/PD-L 1/CTLA-4 inhibitor agent and/or additional anti-cancer agent. In some embodiments, the PPRT will be administered within 5, 4, 3, 2, or 1 days of any treatment with the PD-1/PD-L 1/CTLA-4 inhibitor agent and/or additional anti-cancer agent. It will further be appreciated that the PPRT and the PD-1 /PD-L 1/CTLA-4 inhibitor agent and/or additional anti-cancer agent or treatment may be administered to the subject within a matter of hours or minutes (i.e., substantially simultaneously).

In addition to the administration of the combination of PPRT and at least one PD-1/PD-L 1 inhibitor, it may be useful to also administer additional anticancer agents. Useful classes of anti-cancer agents include, for example, antitubulin agents, auristatins, DNA minor groove binders, DNA replication inhibitors, alkylating agents (e.g., platinum complexes such as cis-platin, mono(platinum), bis(platinum) and tri-nuclear platinum complexes and carboplatin), anthracyclines, antibiotics, antifolates, anti-metabolites, chemotherapy sensitizers, duocarmycins, etoposides, fluorinated pyrimidines, ionophores, lexitropsins, nitrosoureas, platinols, performing compounds, purine anti metabolites, puromycins, radiation sensitizers, steroids, taxanes, topoisomerase inhibitors, vinca alkaloids, or the like. In certain embodiments, the second anti-cancer agent is an antimetabolite, an antimitotic, a topoisomerase inhibitor, or an angiogenesis inhibitor.

Anticancer agents that may be administered in combination with the PPRT and PD-1/PD-L 1/CTLA-4 inhibition include chemotherapeutic agents. Thus, in some embodiments, the method or treatment involves the combined administration of a PPRT and PD-1/PD-L 1/CTLA-4 inhibitor and a chemotherapeutic agent or cocktail of multiple different chemotherapeutic agents. Treatment with a PPRT can occur prior to, concurrently with, or subsequent to administration of these other therapies. Chemotherapies contemplated by the invention include chemical substances or drugs which are known in the art and are commercially available, such as gemcitabine, irinotecan, doxorubicin, 5-fluorouracil, cytosine arabinoside ("Ara-C"), cyclophosphamide, thiotepa, busulfan, cytoxin, TAXOL, methotrexate, cisplatin, melphalan, vinblastine and carboplatin. Combined administration can include co-administration, either in a single pharmaceutical formulation or using separate formulations, or consecutive administration in either order but generally within a time period such that all active agents can exert their biological activities simultaneously. Preparation and dosing schedules for such chemotherapeutic agents can be used according to manufacturers' instructions or as determined empirically by the skilled practitioner.

Chemotherapeutic agents useful in the instant invention also include, but are not limited to, alkylating agents such as thiotepa and cyclosphosphamide (CYTOXAN); alkyl sulfonates such as busulfan, improsulfan, and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylolomelamime nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as aclacinomysins, actinomycin, authramycin, azaserine, bleomycins, cactinomycin, calicheamicin, carabicin, caminomycin, carzinophilin, chromomycins, dactinomycin, daunorubicin, detorubicin, 6-diazo-5-oxo-L-norleucine, doxorubicin, epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-FU; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; amsacrine; bestrabucil; bisantrene; edatraxate; defofamine; demecolcine; diaziquone; elformithine; elliptinium acetate; etoglucid; gallium nitrate; hydroxyurea; lentinan; lonidamine; mitoguazone; mitoxantrone; mopidamol; nitracrine; pentostatin; phenamet; pirarubicin; podophyllinic acid; 2-ethylhydrazide; procarbazine; PSK; razoxane; sizofuran; spirogermanium; tenuazonic acid; triaziquone; 2,2',2"-trichlorotriethylamine; urethan; vindesine; dacarbazine; mannomustine; mitobronitol; mitolactol; pipobroman; gacytosine; arabinoside ("Ara-C"); cyclophosphamide; thiotepa; taxoids, e.g. paclitaxel (TAXOL, Bristol-Myers Squibb Oncology, Princeton, N.J.) and doxetaxel (TAXOTERE, Rhone-Poulenc Rorer, Antony, France); chlorambucil; gemcitabine; 6-thioguanine; mercaptopurine; methotrexate; platinum analogs such as cisplatin and carboplatin; vinblastine; platinum; etoposide (VP-16); ifosfamide; mitomycin C; mitoxantrone; vincristine; vinorelbine; navelbine; novantrone; teniposide; daunomycin; aminopterin; xeloda; ibandronate; CPT11 ; topoisomerase inhibitor RFS 2000; difluoromethylornithine (OMFO); retinoic acid; esperamicins; capecitabine; and pharmaceutically acceptable salts, acids or derivatives of any of the above. Chemotherapeutic agents also include anti-hormonal agents that act to regulate or inhibit hormone action on tumors such as anti-estrogens including for example tamoxifen, raloxifene, aromatase inhibiting 4(5)-imidazoles, 4-hydroxytamoxifen, trioxifene, keoxifene, L Y117018, onapristone, and toremifene (Fareston); and antiandrogens such as flutamide, nilutamide, bicalutamide, leuprolide, and goserelin; and pharmaceutically acceptable salts, acids or derivatives of any of the above.

In certain embodiments, the therapeutic agent is a kinase inhibitor. In certain embodiments, the kinase inhibitor is a multi-targeted receptor tyrosine kinase inhibitor. Kinase inhibitors include, but are not limited to, sunitinib (marketed as Sutent by Pfizer), pazopanib, crizotinib, dasatinib. In certain embodiments, the second anticancer agent is sunitinib.

In certain embodiments, the therapeutic agent is an inhibitor of mammalian target of rapamycin (mTOR). mTOR inhibitors include, but are not limited to, temsirolimus, sirolimus, deforolimus and everolimus. In certain embodiments, the second anticancer agent is everolimus.

In certain embodiments, therapeutic agent is a somatostatin analog. Somatostatin analogs act through interaction with specific, high affinity membrane receptors for somatostatin. Somatostatin analogs include, but are not limited to, octreotide, somatuline, and RC 160 (octastatin). In certain embodiments, the second anticancer agent is octreotide.

In certain embodiments, the chemotherapeutic agent is a topoisomerase inhibitor. Topoisomerase inhibitors are chemotherapy agents that interfere with the action of a topoisomerase enzyme (e.g., topoisomerase I or II). Topoisomerase inhibitors include, but are not limited to, doxorubicin HCl, daunorubicin citrate, mitoxantrone HCl, actinomycin 0, etoposide, Topotecan HCl, teniposide (VM-26), and irinotecan. In certain embodiments, the second anticancer agent is irinotecan.

In certain embodiments, the chemotherapeutic agent is an alkylating agent. In certain embodiments, the chemotherapeutic agent is temozolomide.

In certain embodiments, the chemotherapeutic agent is an anti-metabolite. An anti-metabolite is a chemical with a structure that is similar to a metabolite required for normal biochemical reactions, yet different enough to interfere with one or more normal functions of cells, such as cell division. Anti-metabolites include, but are not limited to, gemcitabine, fluorouracil, capecitabine, methotrexate sodium, ralitrexed, pemetrexed, tegafur, cytosine arabinoside, THIOGUANINE (GlaxoSmithKline), 5-azacytidine, 6-mercaptopurine, azathioprine, 6-thioguanine, pentostatin, fludarabine phosphate, and cladribine, as well as pharmaceutically acceptable salts, acids, or derivatives of any of these. In certain embodiments, the second anticancer agent is gemcitabine. In certain embodiments, the tumor to be treated is a pancreatic neuroendocrine tumor and the second anticancer agent is an anti-metabolite (e.g., gemcitabine).

In certain embodiments, the chemotherapeutic agent is an antimitotic agent, including, but not limited to, agents that bind tubulin. By way of non-limiting example, the agent comprises a taxane. In certain embodiments, the agent comprises paclitaxel or docetaxel, or a pharmaceutically acceptable salt, acid, or derivative of paclitaxel or docetaxel. In certain embodiments, the agent is paclitaxel (TAXOL), docetaxel (TAXOTERE), albumin-bound paclitaxel (e.g., ABRAXANE), DHA-paclitaxel, or PG-paclitaxel. In certain alternative embodiments, the antimitotic agent comprises a vinca alkaloid, such as vincristine, vinblastine, vinorelbine, or vindesine, or pharmaceutically acceptable salts, acids, or derivatives thereof. In some embodiments, the antimitotic agent is an inhibitor of Eg5 kinesin or an inhibitor of a mitotic kinase such as Aurora A or Plk1 .

In certain embodiments, the treatment involves the combined administration of a PPRT and a PD-1/PD-L 1/CTLA-4 inhibitor described herein and radiation therapy. Treatment with the PPRT can occur prior to, concurrently with, or subsequent to administration of radiation therapy. Any dosing schedule for such radiation therapy can be used as determined by the skilled practitioner.

In some embodiments, the second anti-cancer agent comprises an antibody. Thus, treatment can involve the combined administration of a PPRT and the PD-1/PD-L 1/CTLA-4 inhibitor agent with antibodies against tumor-associated antigens including, but not limited to, antibodies that bind to EGFR, ErbB2, HER2, DLL4, Notch and/or VEGF. In certain embodiments, the second anti-cancer agent is an antibody that is an angiogenesis inhibitor (e.g., an anti-VEGF antibody). In certain embodiments, the second anti-cancer agent is an inhibitor of Notch signaling. In certain embodiments, the second anti-cancer agent is AVASTIN (Bevacizumab), Herceptin (Trastuzumab), VECTIBIX (Panitumumab), or Erbitux (Cetuximab). Combined administration can include co-administration, either in a single pharmaceutical formulation or using separate formulations, or consecutive administration in either order but generally within a time period such that all active agents can exert their biological activities simultaneously.

Furthermore, treatment can include administration of one or more cytokines (e.g., lymphokines, interleukins, tumor necrosis factors, and/or growth factors) or can be accompanied by surgical removal of cancer cells or cytoreductive treatments such as (chemo-radio)embolization, radiofrequency ablation and high-intensity focused ultrasound (HIFU) ablation or any other therapy deemed necessary by a treating physician.

For the treatment of the disease, the appropriate dosage of a described herein depends on the type of neuroendocrine tumor to be treated, the severity and course of the neuroendocrine tumor, the responsiveness of the neuroendocrine tumor, whether the PPRT and the PD-1/PD-L 1/CTLA-4 is administered for therapeutic or preventative purposes, previous therapy, patient's clinical history, and so on all at the discretion of the treating physician. The PPRT and the PD-1/PD-L 1/CTLA-4 inhibitor can be administered one time or over a series of treatments lasting from several days to several months, or until a cure is effected or a diminution of the neuroendocrine tumor is achieved (e.g. reduction in tumor size). Optimal dosing schedules can be calculated from measurements of drug accumulation in the body of the patient and will vary depending on the relative potency of an individual dosing regimen.

The administering physician can easily determine optimum dosages, dosing methodologies and repetition rates. In certain embodiments, dosage is from 0.01 .mu.g to 100 mg per kg of body weight, and can be given once or more daily, weekly, monthly or yearly. In certain embodiments, the PPRT and the PD-1/PD-L 1/CTLA-4 inhibitor agent is given once every two weeks or once every three weeks. In certain embodiments, the dosage of the PPRT and the PD-1 /PD-L 1/CTLA-4 inhibitor agent is from about 0.1 mg to about 20 mg per kg of body weight. The treating physician can estimate repetition rates for dosing based on measured residence times and concentrations of the drug in body fluids or tissues.

Usually PRRT with Lutathera is given in a cycles wherein each cycle comprises 3.7-7.4GBq administered intravenously. Typically, a therapeutic cumulative activity (dose) of approximately 30 GBq may readily be divided in 4 to 6 cycles, each administered every 5 to 12 weeks. It should be understood that longer intercycle intervals as long as 6 Months or even years can be used. Moreoever, the patient may be treated with multiple rounds of the 4 to 6 cycles after months or years from the first set of treatments. The second and subsequent set of treatments may use the same activities (doses) or may use different activities (doses) depending on the experience of the center and of the characteristics and therapeutic needs of each patient.

Anti-PD-1 nivolumab is typically administered intravenously at doses of about 3 mg/kg every 2-3 weeks, for an initial period of two years. Thereafter, maintenance therapies every 12 weeks after the initial treatment are frequently used. It should be understood that these dosing regimens will vary according to the patient's response to the treatments and at the discretion of the treating clinician. The recommended dose of ipilimumab for the treatment of unresectable or metastatic melanoma is 3 mg/kg administered intravenously over 90 minutes every 3 weeks for a total of four doses.

The PPRT and the PD-1/PD-L 1/CTLA-4 inhibitors (e.g., antibodies and soluble receptors) can be formulated into a pharmaceutical composition by any suitable method known in the art. In certain embodiments, the pharmaceutical compositions comprise a pharmaceutically acceptable vehicle. The pharmaceutical compositions find use in inhibiting neuroendocrine tumor growth and treating neuroendocrine tumor in human patients.

### Example

**Example** 1: Lutathera is administered at a fixed dose of 7.4 GBq (every 12 weeks) up to a cumulative dose which is tolerated by the patient (maximum 29.6 GBq). Nivolumab is administered twice for each Lutathera treatment at the dose of 3 mg/Kg: one administration seven days before (d-7) and the other administration seven days after (d+7) administration of Lutathera with the aim of achieving an effective PD-1/PD-L1 blockade, but also in the need not to overlap the anticipated lymphocyte nadir related to the lymphocytopenia-induced effect of Lutathera.
Studies have shown that intravenous administration of amino acids has a renal protective effect. An infusion of amino acids (containing lysine and arginine) could be done 30 to 45 minutes before the administration of 177Lu-DOTATATE and last for 3 to 4 hours.

### REFERENCES

1 Ricci S. Long-acting depot lanreotide in the treatment of patients with advanced neuroendocrine tumors. Am. J. Clin. Oncol. 23(4),412-415 (2000).
2 Wynick D. Resistance of metastatic pancreatic endocrine tumours after longterm treatment with the somatostatin analogue octreotide (SMS 201-995). Clin. Endocrinol. 30(4), 385-388 (1989).
3 Wynick D. The use of the long-acting somatostatin analog octreotide in the treatment of gut neuroendocrine tumors. J. Clin. Endocrinol. Metab. 73(1), 1-3 (1991).
4 Strosberg JR. First-line chemotherapy with capecitabine and temozolomide in patients with metastatic pancreatic endocrine carcinomas. Cancer 001: 10.1 002/cncr.25425 (2010)
5 Pardoll OM. Nat Rev Cancer. 2012;12(4):252-264.
6 Sharma P, Wagner K, Wolchok JD, Allison JP. Nat Rev Cancer. 2011 ;11 (11 ):805-812.
7 Schultei et al. , PD-L 1 expression in small cell neuroendocrine carcinomas, Eur J Cancer, 2015 Feb;51 (3):421-6. doi: 10.1 016/j.ejca.2014.12.006. Epub 2015 Jan 9.
8 Grabowski P, Joehrens K, Arsenic R, et al. Tumor infiltrating lymphocytes and PD-L 1 Epression Differ in Low and High grade Neuroendocrine Tumors. Abstract H11, presented at ENETS meeting, Barcelona 2015
9 Formenti S. et al. J.Natl.Cancer Inst., 105(4): 256-265, 2013
10 Bernstein M. et al. Cancer Biotherapy and Radiopharmaceuticals, 29(4): 153161, 2014).
11 Berbstein M. and al., Cancer Biotherapy and radiopharmaceuticals, 29(4), 153-161, 2014
12 Wu Y. et al. Diagnostics, 3, 344-355, 2013
13 Denoyer D, Lobachevsky P, Jackson P, Thompson M, Martin OA, Hicks RJ. Analysis of 177Lu-DOTA-octreotate therapy-induced DNA damage in peripheral blood lymphocytes of patients with neuroendocrine tumors J Nucl Med. 2015 Apr;56(4):505-11.
14. Linnebacher M1, Maletzki C. Tumor-infiltrating B cells: The ignored players in tumor immunology. Oncoimmunology. 2012 Oct 1;1(7):1186-1188.
15. Nelson BH. CD20+ B cells: the other tumor-infiltrating lymphocytes. J Immunol. 2010 Nov 1;185(9):4977-82.
16 Sierra ML, Agazzi A, Bodei L, Pacifici M, Aricò D, De Cicco C, Quarna J, Sansovini M, De Simone M, Paganelli Lymphocytic toxicity in patients after peptide-receptor radionuclide therapy (PRRT) with 177Lu-DOTATATE and 90Y-DOTATOC. Cancer Biother Radiopharm. 2009 Dec;24(6):659-65.
17 Imhof A, et al., Response, Survival, and Long-Term Toxicity After Therapy With the Radiolabeled Somatostatin Analogue [90Y-DOTA]-TOC in Metastasized Neuroendocrine CancersJ Clin Oncol, 2011;29:2416-231
18 Phan AT, Oberg K, Choi J, Harrison LH Jr, Hassan MM, Strosberg JR, Krenning EP, Kocha W, Woltering EA, Maples WJ; North American Neuroendocrine Tumor Society (NANETS). NANETS consensus guideline for the diagnosis and management of neuroendocrine tumors: well-differentiated neuroendocrine tumors of the thorax (includes lung and thymus). Pancreas. 2010 Aug;39(6):784-98.
19. Schwartz RH. Costimulation of T lymphocytes: the role of CD28, CTLA-4, and B7/BB1 in interleukin-2 production and immunotherapy. Cell. 1992;71:1065-8.
20. Krummel MF, Allison JP. CD28 and CTLA-4 have opposing effects on the response of T cells to stimulation. J Exp Med. 1995;182:459-65.
21. Linsley PS, Brady W, Urnes M, et al. CTLA-4 is a second receptor for the B cell activation antigen B7. J Exp Med. 1991;174:561-9.
22. Krummel MF, Allison JP. CTLA-4 engagement inhibits IL-2 accumulation and cell cycle progression upon activation of resting T cells. J Exp Med. 1996;183:2533-40.
23. Wing K, Onishi Y, Prieto-Martin P, et al. CTLA-4 control over Foxp3+ regulatory T cell function. Science. 2008;322:271-5.
24. Peggs KS, Quezada SA, Chambers CA, et al. Blockade of CTLA-4 on both effector and regulatory T cell compartments contributes to the antitumor activity of anti-CTLA-4 antibodies. J Exp Med. 2009;206:1717-25.
25. Simpson TR, Li F, Montalvo-Ortiz W, et al. Fcdependent depletion of tumor-infiltrating regulatory T cells co-defines the efficacy of anti-CTLA-4
26. Slovin SF, Higano CS, Hamid O, Tejwani S, Harzstark A, Alumkal JJ, et al. Ipilimumab alone or in combination with radiotherapy in metastatic castration-resistant prostate cancer: results from an open-label, multicenter phase I/II study. Ann Oncol 2013;24:1813-21.
27. Kwon ED, Drake CG, Scher HI, Fizazi K, Bossi A, van den Eertwegh AJ, et al. Ipilimumab versus placebo after radiotherapy in patients with metastatic castration-resistant prostate cancer that had progressed after docetaxel chemotherapy (CA184-043): a multicentre, randomised, doubleblind, phase 3 trial. Lancet Oncol 2014;15:700-12.
28. Grimaldi AM, Simeone E, Giannarelli D, et al. The abscopal effect: efficacy of radiotherapy in patients on progression after treatment with ipilimumab 3 mg/kg. Journal for Immunotherapy of Cancer. 2013;1(Suppl 1):O23.
29. Antonia SJ, Gettinger SN, Chow LQM, et al. Nivolumab and ipilimumab in first-line NSCLC: interim phase I results. J Clin Oncol. 2014;32(5s suppl; abstr 8023).
30. Antonia SJ, Goldberg S, Balmanoukian AS, et al. Phase Ib study of MEDI4736, a programmed cell death ligand-1 (PD-L1) antibody, in combination with tremelimumab, a cytotoxic T-lymphocyte-associated protein-4 (CTLA-4) antibody, in patients (pts) with advanced NSCLC. J Clin Oncol. 2015;33(suppl; abstr 3014).
31. Bodei L, Kidd M et al. (2014). Long-term tolerability of PRRT in 807 patients with neuroendocrine tumours: the value and limitations of clinical factors. Eur J Nucl Med Mol Imaging, ahead of publication, DOI 10.1007/s00259-014-2893-5

### FEATURES

1. A method of treating a patient that has a cancer that over-expresses somatostatin receptors comprising administering to said patient a combination of a Peptide Receptor Radionuclide therapy (PRRT) and an Immuno-oncology therapy, wherein the combined effect of the PRRT and the Immuno-oncology therapy, produces a therapeutic effect on said cancer.
2. A method of treating a patient that has a cancer that over-expresses somatostatin receptors comprising administering to said patient a combination of a Peptide Receptor Radionuclide therapy (PRRT) and an inhibitor that inhibits the PD-1 /PD-L 1 /CTLA-4 pathway, wherein the combined effect of the PRRT and the inhibitor of PD-1 IPD-L 1 pathway produces a therapeutic effect on said cancer.
3. The method of feature 1, wherein said PRRT is [177]Lutetium-DOTA[O]- Tyr[3]-Octreotate.
4. The method of feature 1 , wherein said inhibitor of the PD-1 /PD-L1 /CTLA-4 pathway is an antibody that targets PD-L 1 .
5. The method of feature 1 wherein said inhibitor of the PD-1 /PD-L 1 pathway is an antibody that targets PD-1.
6. The method of feature 1 wherein said inhibitor of the the PD-1 IPD-L 1 pathway is selected from the group consisting of Nivolumab, MK-3475, MPDL3280A, MED14736, ipilimumab, and tremelimumab..
7. The method of feature 1 , wherein said cancer is a neuroendocrine tumor.
8. The method of feature 6, wherein said treating comprises one or more of inhibiting the growth of a neuroendocrine tumor, inhibiting proliferation of neuroendocrine tumor cells, inhibiting neuroendocrine tumor metastases, inducing neuroendocrine tumor cell differentiation, reducing tumorgenicity of neuroendocrine tumor cells and methods of reducing the frequency of cancer stem cells or tumor initiating cells in a neuroendocrine tumor.
9. The method of feature 6, wherein said neuroendocrine tumor is selected from the group consisting of a gastroenteropancreatic neuroendocrine tumor, carcinoid tumor, pheochromocytoma, paraganglioma, medullary thyroid cancer, pulmonary neuroendocrine tumor, thymic neuroendocrine tumor, a carcinoid tumor or a pancreatic neuroendocrine tumor, pituitary adenoma, adrenal gland tumors, Merkel cell carcinoma, breast cancer, Non-Hodgkin lymphoma, Hodgkin lymphoma, Head & Neck tumor, urothelial carcinoma (bladder), Renal Cell Carcinoma, Hepatocellular Carcinoma, GIST, neuroblastoma, bile duct tumor, cervix tumor, Ewing sarcoma, osteosarcoma, SCLC, prostate cancer, melanoma, meningioma, glioma, medulloblastoma hemangioblastoma, supratentorial primitive, neuroectodermal tumor, and esthesioneuroblastoma..
10. The method of feature 6, wherein said neuroendocrine tumor is selected from the group consisting of functional carcinoid tumor, insulinoma, gastrinoma, vaso active intestinal peptide (VI P)oma, glucagonoma, serotoninoma, histaminoma, ACTHoma, pheocromocytoma, and somatostatinoma.
11. The method of feature 6, wherein said neuroendocrine tumor is a low grade, medium grade, or high grade neuroendocrine tumor.
12. The method of feature 1, wherein said cancer is a small cell lung cancer.
13. The method of feature 1, wherein said cancer is a progressive midgut neuroendocrine tumor.
14. The method of feature 12, wherein said cancer is not responsive to Sandostatin (Novartis) or Somatuline^{®} (Ipsen).
15. The method of feature 1, wherein said cancer is non-responsive or has a low response to an inhibitor of the PD-1 /PD-L1 pathway.
16. The method of feature 6, wherein said neuroendocrine tumor is a functional neuroendocrine tumor.
17. The method of feature 6, wherein said neuroendocrine tumor is a nonfunctional neuroendocrine tumor.

## Claims

1. A radiolabeled somatostatin analogue peptide for use in the treatment of a cancer that over-expresses somatostatin receptors in a patient in need thereof, wherein said radiolabeled somatostatin analogue peptide is administered to said patient, as Peptide Receptor Radionuclide Therapy (PRRT) in combination with an inhibitor that inhibits the PD-1 /PD-L 1 /CTLA-4 pathway.

2. A radiolabeled somatostatin analogue peptide for use according to claim 1, wherein said radiolabeled somatostatin analogue peptide is [177]Lutetium-DOTA[O]- Tyr[3]-Octreotate or 177Lu-DOTATOC.

3. A radiolabeled somatostatin analogue peptide for use according to claim 1 or 2, wherein said inhibitor of the PD-1 /PD-L1 /CTLA-4 pathway is an antibody that targets PD-L 1

4. A radiolabeled somatostatin analogue peptide for use according to claim 1 or 2 wherein said inhibitor of the PD-1 /PD-L 1 /CTLA-4 pathway is an antibody that targets PD-1.

5. A radiolabeled somatostatin analogue peptide for use according to any one of claims 1 to 4, wherein said inhibitor of the PD-1 /PD-L 1 /CTLA-4 pathway is selected from the group consisting of Nivolumab, MK-3475, MPDL3280A, MED14736, ipilimumab, and tremelimumab.

6. A radiolabeled somatostatin analogue peptide for use according to any one of claims 1 to 5, wherein said cancer is a neuroendocrine tumor.

7. A radiolabeled somatostatin analogue peptide for use according to any one of claims 1 to 6, wherein said treatment comprises one or more of inhibiting the growth of a neuroendocrine tumor, inhibiting proliferation of neuroendocrine tumor cells, inhibiting neuroendocrine tumor metastases, inducing neuroendocrine tumor cell differentiation, reducing tumorgenicity of neuroendocrine tumor cells and methods of reducing the frequency of cancer stem cells or tumor initiating cells in a neuroendocrine tumor.

8. A radiolabeled somatostatin analogue peptide for use according to claim 6, wherein said neuroendocrine tumor is selected from the group consisting of a gastroenteropancreatic neuroendocrine tumor, carcinoid tumor, pheochromocytoma, paraganglioma, medullary thyroid cancer, pulmonary neuroendocrine tumor, thymic neuroendocrine tumor, a carcinoid tumor or a pancreatic neuroendocrine tumor, pituitary adenoma, adrenal gland tumors, Merkel cell carcinoma, breast cancer, Non-Hodgkin lymphoma, Hodgkin lymphoma, Head & Neck tumor, urothelial carcinoma (bladder), Renal Cell Carcinoma, Hepatocellular Carcinoma, GIST, neuroblastoma, bile duct tumor, cervix tumor, Ewing sarcoma, osteosarcoma, SCLC, prostate cancer, melanoma, meningioma, glioma, medulloblastoma hemangioblastoma, supratentorial primitive, neuroectodermal tumor, and esthesioneuroblastoma.

9. A radiolabeled somatostatin analogue peptide for use according to claim 6, wherein said neuroendocrine tumor is selected from the group consisting of functional carcinoid tumor, insulinoma, gastrinoma, vaso active intestinal peptide (VI P)oma, glucagonoma, serotoninoma, histaminoma, ACTHoma, pheocromocytoma, and somatostatinoma.

10. A radiolabeled somatostatin analogue peptide for use according to claim 6, wherein said neuroendocrine tumor is a low grade, medium grade, or high grade neuroendocrine tumor.

11. A radiolabeled somatostatin analogue peptide for use according to any one of claims 1 to 5, wherein said cancer is a small cell lung cancer.

12. A radiolabeled somatostatin analogue peptide for use according to any one of claims 1 to 5, wherein said cancer is a progressive midgut neuroendocrine tumor.

13. A radiolabeled somatostatin analogue peptide for use according to any one of claims 1 to 5, wherein said cancer is non-responsive or has a low response to an inhibitor of the PD-1 /PD-L 1 /CTLA-4pathway.

14. A radiolabeled somatostatin analogue peptide for use according to claim 6, wherein said neuroendocrine tumor is a functional neuroendocrine tumor.

15. A radiolabeled somatostatin analogue peptide for use according toclaim 6, wherein said neuroendocrine tumor is a nonfunctional neuroendocrine tumor.
